# EUROPÄISCHE PATENTSCHRIFT

(11) **EP 1 796 626 B1**
(45) Veröffentlichungstag und Bekanntmachung des Hinweises auf die Patenterteilung: **21.07.2010**
(21) Anmeldenummer: 05792053.0
(22) Anmeldetag: 27.09.2005
(51) Int. Cl.: A61K 9/00, A61K 47/26

(54) **NEUE PULVERINHALATIVA AUF BASIS MODIFIZIERTER LAKTOSEMISCHUNGEN ALS HILFSSTOFF**
NOVEL POWDER INHALATION PREPARATIONS BASED ON MODIFIED LACTOSE BLENDS THAT ARE USED AS ADJUVANTS
POUDRES A INHALER A BASE DE MELANGES DE LACTOSES MODIFIES COMME AUXILIAIRES

(30) Priorität: 01.10.2004 DE 102004048390
(43) Veröffentlichungstag der Anmeldung: 20.06.2007
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: TRUNK, Michael, 55218 INGELHEIM (DE); MUELLER, Bernd, Wilhelm, 24220 FLINTBEK (DE); STECKEL, Hartwig, Andreas, 24232 SCHOENKIRCHEN (DE); TIMMERMANN, Inga-Lis, 13156 BERLIN (DE)
(74) Vertreter: Hammann, Heinz
(86) Internationale Anmeldenummer: PCT/EP2005/054833
(87) Internationale Veröffentlichungsnummer: WO 2006/037736

(56) Entgegenhaltungen:
- WO-A-01/76560
- WO-A-02/07705
- BUCKTON GRAHAM ET AL: "The use of gravimetric studies to assess the degree of crystallinity of predominantly crystalline powders" INTERNATIONAL JOURNAL OF PHARMACEUTICS (AMSTERDAM), Bd. 123, Nr. 2, 1995, Seiten 265-271, XP002365264 ISSN: 0378-5173
- NAINI V ET AL: "PHYSICOCHEMICAL STABILITY OF CRYSTALLINE SUGARS AND THEIR SPRAY-DRIED FORMS: DEPENDENCE UPON RELATIVE HUMIDITY AND SUITABILITY FOR USE IN POWDER INHALERS" DRUG DEVELOPMENT AND INDUSTRIAL PHARMACY, NEW YORK, NY, US, Bd. 24, Nr. 10, 1998, Seiten 895-909, XP008003519 ISSN: 0363-9045
- DILWORTH S E ET AL: "Approaches to determine the enthalpy of crystallisation, and amorphous content, of lactose from isothermal calorimetric data" INTERNATIONAL JOURNAL OF PHARMACEUTICS, AMSTERDAM, NL, Bd. 284, Nr. 1-2, 11. September 2004 (2004-09-11), Seiten 83-94, XP004580740 online ISSN: 0378-5173
- STECKEL H ET AL: "Calorimetric study on amorphous lactose during and after re-crystallization at different relative humidity" PHARMAZEUTISCHE INDUSTRIE 2005 GERMANY, Bd. 67, Nr. 6, 2005, Seiten 709-712, XP009060674 ISSN: 0031-711X

## Beschreibung

Die Erfindung betrifft ein neues Herstellverfahren zur Darstellung modifizierter Laktosemischungen, diese Laktosemischungen als solche sowie Zusammensetzung von Arzneimitteln zur Pulverinhalation bestehend aus einem oder mehreren mikronisierten Wirkstoffen und der erfindungsgemäß modifizierten Laktose.

### Hintergrund der Erfindung

Wirkstoffe, die topisch inhalativ verfügbar sein sollen, werden üblicherweise mittels oraler inhalativer Applikation zugänglich gemacht. Sofern sich dieser Weg aufgrund spezieller Eigenschaften des Wirkstoffes oder besonderer Anforderungen an die Applikation als nicht direkt umsetzbar erweist, sind bei der technischen Umsetzung entsprechende weitere und Wirk- und Hilfsstoff-spezifische Maßnahmen anzuwenden, so dass in Abhängigkeit der physiko-chemischen Substanzeigenschaften ein stabiles Produkt reproduzierbar technisch herstellbar ist. Für Substanzen, die sich aufgrund ihres Zersetzungsverhaltens in Lösung als kritisch erweisen, bietet sich die Pulverinhalation an. Dabei stellt sowohl die absolute Menge dieses Wirkstoffes, die bei einer Anwendung verabreicht werden muss, eine besondere Herausforderung an die Formulierung dar, als auch erweist sich die physikalische Stabilität (z.B. aerodynamische Partikelgröße, Dispergierbarkeit, physikochemische Eigenschaften) des mikronisierten Wirkstoffes als kritische Herausforderung zur Entwicklung und Herstellung eines Pulverinhalativums.

Bei der Applikationsform Pulverinhalativum werden Inhalationspulver, die beispielsweise in geeignete Kapseln (Inhaletten) abgefüllt werden, mittels Pulverinhalatoren ausgebracht. Ebenso sind weitere Systeme, in denen die zu applizierende Pulvermenge vordosiert ist (z.B. Blister), als auch Multidose Pulversysteme bekannt. Alternativ dazu kann eine inhalative Anwendung auch durch Applikation geeigneter pulverförmiger Inhalationsaerosole, die beispielsweise in HFA134a, HFA227 oder deren Gemisch als Treibgas suspendiert sind, erfolgen.

Üblicherweise werden Pulverinhalativa, z.B. in Form von Kapseln zur Inhalation, auf Basis der allgemeinen Lehre, wie sie in DE-A-179 22 07 beschrieben ist, hergestellt. Ein weiterer bedeutender Aspekt bei der Entwicklung von Pulverinhalativa ist, dass bei der inhalativen Applikation des Wirkstoffes nur Teilchen einer bestimmten aerodynamischen Größe in das Zielorgan Lunge gelangen. Die Teilchengröße dieser lungengängigen Partikel (inhalierbarer Anteil) liegt im Bereich weniger µm, typischerweise zwischen 1 und 10µm, vorzugsweise unter 6µm. Solche Partikel werden üblicherweise durch Mikronisierung (z.B. Luftstrahlmahlung) erzeugt.

Es hat sich dabei herausgestellt, dass die Optimierung von Pulverinhalativa u.a. nur dadurch erreichbar ist, dass in Abhängigkeit der Eigenschaften des Wirkstoffes aus den auf dem Markt angebotenen Laktosen jeweils entsprechend den gewünschten Produkteigenschaften die geeignetste ausgewählt wird. Entsprechend den Bedürfnissen werden beispielsweise von Laktose-Herstellern diverse Qualitäten angeboten. Zur Verbesserung der Produkteigenschaften von Pulverinhalativa sind in der Zwischenzeit auch Möglichkeiten vorgeschlagen worden, wie käuflich erwerbbare Laktosen für die speziellen Einsatzzwecke im Rahmen der Produktherstellung modifiziert und optimiert werden können (z.B.: DE 4140689).

Es stellt sich jedoch weiterhin dar, dass die gegebenen morphologischen und kristallinen Eigenschaften (in erster Linie an der Partikeloberfläche) der eingesetzten Laktose maßgeblich zu den Produkteigenschaften beitragen.

Pulverinhalativa, wie sie nach dem Stand der Technik hergestellt werden können (beispielsweise DE-A-179 22 07), müssen neben der chemischen Stabilität des Wirkstoffes und der Zusammensetzung des Arzneimittels als solche vor allem auch physikalisch konstante Eigenschaften aufweisen. Darunter versteht man in direkter Art und Weise, dass die Aerosoleigenschaften hinsichtlich ihres aerodynamischen Verhaltens (z. B. aerodynamische Partikelgröße) konstant bleiben. Dies bedingt, dass sowohl für den mikronisierten Wirkstoff als auch für die im Produkt eingesetzten Hilfsstoffe diese in ihrer physiko-chemisch stabilsten Form eingesetzt werden sollten. Für Laktose gilt dabei aus thermodynamischer Sicht, dass dies dann gegeben ist, wenn sie unter Normalbedingungen in Form von alpha-Laktose Monohydrat vorliegt. Dies gilt in besonderem Maße für die kristalline Ordnung der Oberfläche der Partikel.

Es ist bekannt, dass bei der Mahlung von organischen Substanzen durch den starken Eintrag mechanischer Energie amorphe Anteile bzw. Veränderungen der kristallinen Struktur zu thermodynamisch instabileren Modifikationen auftreten können.

Darüber hinaus ist bekannt, dass Lactose in diversen Formen vorkommen kann (Coenraad F. Lerk, Physikalisch-pharmazeutische Eigenschaften von Lactose, Pharmazie in unserer Zeit, 16, 1987, Nr. 2; 39-46).

Beispielsweise während der Mahlung von Laktose oder bei sonstiger mechanischer Belastung entstehende amorphe Laktose kann mittels bekannter technischer Verfahren (z.B. WO 92/18110 oder WO 95/05805) rekristallisiert werden. Diese Verfahren zeigen jedoch den Mangel auf, dass ausschließlich der Abbau amorpher Anteile, wie sie nach Mikronisierverfahren auftreten, erreicht werden kann. Diese Verfahren bedingen jedoch nicht zwingend den Abbau von beta-Laktose, welche bei diesen Konditionierverfahren, sowie bei großtechnischen Mahlungen von Laktose auf einen Korngrößenbereich mit einem Wert x₅₀> 10 µm ebenfalls entstehen können. Thermodynamisch instabile Anteile, wie beispielsweise beta-Laktose, stellen auch wenn sie lediglich auf der Oberfläche von Laktose vorhanden sind, einen Faktor dar, welcher beispielsweise die Langzeit- und Lagerstabilität eines Inhalationspulvers negativ beeinflussen kann. Selbst nach Anwendung spezieller Verfahren zur Rekristallisation von amorphen Anteilen an Laktose, wie sie im Stand der Technik beschrieben sind (siehe dazu z.B. WO 92/18110 oder WO 95/05805), erhält man Partikel, die trotz Rekristallisation von gegebenenfalls vorhandenen amorphen Anteilen weiterhin eine Mikrostrukturierung auf den Partikeln erkennen lassen, welche mit Bereichen erhöhter Oberflächen-Energie gleichgesetzt werden kann.

In WO 01/76560 wurde ein Verfahren zur Herstellung eines Trägermaterials offenbart, bei dem die Kleinpartikelfraktion abgetrennt wurde.

In WO 02/07705 wurde ein Verfahren zur Herstellung eines Trägermaterials offenbart, welches sphärische Partikel, jedoch keinerlei amorphen Bereiche enthält.

Es ist Aufgabe der vorliegenden Erfindung, ein Verfahren bereitzustellen, durch das Laktosepartikel derartig modifiziert werden, dass diese einerseits (primär an der Oberfläche) eine hochkristalline Textur der thermodynamisch stabilsten Modifikation unter Normalbedingungen, der alpha-Laktose Monohydrat, aufweisen und dabei Partikeloberflächen derartig sind, dass diese als äußerst glatt und weitestgehend frei von einer Mikrostruktur beschrieben werden können.

### Beschreibung der Erfindung

Die durch die vorliegende Erfindung zur Lösung der vorstehend genannten Aufgabe vorgeschlagene Verwendung einer Laktosemischung als Hilfsstoff bei der Herstellung eines wirkstoffhaltigen Inhalationspulvers ist dadurch gekennzeichnet, dass die Laktosemischung hergestellt wird durch ein Verfahren, bei dem in einem ersten Schritt eine Trägerlaktose mit einer mittleren Teilchengröße, gemessen mit einem Laserdiffraktometer nach der Trockendispersionsmethode von X₅₀ = 17-50µm einer Laktosefraktion mit einer geringeren mittleren Teilchengröße, gemessen mit einem Laserdiffraktometer nach der Trockendispersionsmethode von X₅₀< 25µm zugemischt wird, wobei die Laktosefraktion mit der geringeren mittleren Teilchengröße einen hohen Anteil amorphes Laktose enthält, und in einem Zweiten Schritt die so erhaltene Laktosemischung einer Relation Luftfeuchte von wenigstens 85% r.F. bei einer Temperatur von 20°C-40°C für einen Zeitraum von wenigstens 4 Stunden ausgesetzt wird, und in einem dritten Schritt die erhaltene Laktosemischung einem Trocknungsschritt ausgesetzt wird, wobei die Laktose einer Luftfeuchte kleiner 20% r.F. unter Erhöhung der Temperatur für einen Zeitraum von mindestens 1 Stunde ausgesetzt wird. Während des Mischprozesses der Vormischung (der beiden Laktosequalitäten) können sich die mikronisierten, amorphen Laktosepartikel durch Herstellung einer interaktiven Mischung auf der Trägerlaktose anlagern. Durch gezielte Rekristallisation der amorphen Laktose bei erhöhter relativer Luftfeuchte können dann die Oberflächenrauhigkeit, Unebenheiten, auftretende Bruchkanten, Poren und energetisch aktivierte Stellen (Hot Spots) der Trägerlaktose bedeckt werden, um so eine modifizierte Trägerlaktose zu generieren, durch die Adhäsionskraft zwischen Träger und dem in einem weiteren Schritt zu zu mischenden mikronisierten Wirkstoff gezielt verringert und stabilisiert werden kann.

Unter dem Medianwert X₅₀ versteht man die Teilchengröße, unterhalb derer 50% der Teilchenmenge liegt, gemessen mit einem Laserdiffraktometer nach der Trockendispersionsmethode. Diese Methode ist im Stand der Technik bekannt und wird beispielsweise durch die WO 02/30389 offenbart.

In einer bevorzugten Ausführungsform des erfindungsgemäßen Verfahrens wird die Laktosemischung der genannten relativen Feuchte für einen Zeitraum von wenigstens 6 Stunden, besonders bevorzugt wenigstens 12 Stunden, ferner bevorzugt wenigstens 18 Stunden ausgesetzt.

Im Rahmen des erfindungsgemäßen Verfahrens erfolgt das Einwirken der Feuchtigkeit bevorzugt bei einer Temperatur von 25-35°C, beispielsweise bei 25°C, 30°C oder 35°C.

In einer bevorzugten Ausführungsform des erfindungsgemäßen Verfahrens, wird die Laktosemischung einer relativen Feuchte von wenigstens 90% r.F., besonders bevorzugt von wenigstens 93% r.F. ausgesetzt.

Unter relativer Feuchte (r.F.) wird in Übereinstimmung mit dem Stand der Technik auch im Rahmen der vorliegenden Erfindung der Quotient des Partialdruckes des Wasserdampfs und dem Dampfdruckes des Wassers bei der betreffenden Temperatur verstanden.

In bevorzugten Ausführungsformen des erfindungsgemäßen Verfahrens wird ferner so vorgegangen, dass
a) die Laktosemischung derart bereitgestellt wird, dass eine möglichst große Pulverbett-Oberfläche ausgebildet wird,
b) die Laktosemischung sodann den Bedingungen des Konditionierungsschritts gemäss Anspruch 1 ausgesetzt wird;
c) und dann bei dem Trocknungsschritt gemäss Anspruch 1 eine Reduzierung der relativen Luftfeuchte, welcher das Laktose-Muster ausgesetzt wird, erfolgt.
Bevorzugt wird erst nach Durchführung der Schritte a und b die Pulverbettoberfläche reduziert. Im Verfahrensschritt c wird hierbei unter erhöhter Temperatur die relative Luftfeuchte auf einen Wert kleiner 20% r.h. beziehungsweise kleiner 10% r.h. gebracht.

Während des Trocknungsschitts des erfindungsgemäßen Verfahrens wird überschüssiges Prozesswasser aus dem System entfernt. Dabei kann die Trocknung durch Reduzierung der relativen Feuchte auf kleiner 20%, bevorzugt kleiner 15 % unter Erhöhung der Temperatur (beispielsweise auf 70°C, oder 75°C oder 80°C oder 85°C) in Abhängigkeit einer Prozesszeit von mindestens 1h, bevorzugt mindestens von 2 h erfolgen. In einer Variante kann das zu trocknende Laktoseprodukt auch einer absolut trockenen Umgebung, wahlweise trockenem Stickstoff mit einer Restfeuchte kleiner 5% r.F. ausgesetzt werden, wobei dann Prozesszeiten von mindestens 6 h, bevorzugt mindestens 12 h gewählt werden sollten.

Besonders bevorzugt ist ferner vorstehend genanntes Verfahren, in dem im ersten Schritt als Laktose mit geringerer mittlere Teilchengröße und hohem amorphen Anteil eine nach im Stand der Technik bekannten Verfahren sprühgetrocknete Laktose eingesetzt wird.

Besonders bevorzugt ist ferner vorstehend genanntes Verfahren in dem Trägerlaktose mit einer mittleren Teilchengröße x₅₀ von 20 bis 30µm eingesetzt wird. Besonders bevorzugt ist ferner vorstehend genanntes Verfahren, in dem die vorzugsweise sprühgetrocknete Lactose mit geringerer mittlerer Teilchengröße eine mittlere Teilchengröße x₅₀ < 25µm aufweist. Besonders bevorzugt weist letztgenannte Lactose einen Wert x₉₀ < 100µm, besonders bevorzugt < 70µm auf.

Bevorzugt ist ferner das erfindungsgemäße Verfahren, in dem der Anteil der im ersten Schritt zugemischten, bevorzugt sprühgetrockneten feineren Laktose an der Gesamthilfsstoffrnenge 0,1 bis 20%, bevorzugt 1 bis 15%, besonders bevorzugt 1,5 bis 10%, ferner bevorzugt 2 bis 5 % beträgt.

Ein weiterer Aspekt betrifft die obigem Verfahren erhältlichen Laktosemischungen als solche. Die vorliegende Erfindung betrifft die Verwendung von nach dem erfindungsgemäßen Verfahren erhältlicher Laktosemischung als Hilfsstoff bei der Herstellung eines wirkstoffhaltigen Inhalationspulvers.

Die Mischung der Laktosebestandteile gemäß dem ersten Schritt des erfindungsgemäßen Verfahrens als auch die Mischung der danach erhaltenen Laktosemischungen mit einem geeigneten Wirkstoff können nach im Stand der Technik bekannten Verfahren erhalten werden (z.B. WO 02/30390, WO 03/017970 oder auch WO 03/017979).

Die mittels des erfindungsgemäßen Herstellverfahrens erhältliche Laktose kann generell als Hilfsstoff zur Herstellung von Inhalationspulvern eingesetzt werden, die Wirkstoffe enthalten, deren Applikation aus therapeutischer Sicht auf inhalativem Wege sinnvoll erscheint. Bevorzugt gelangen hierbei solche Wirkstoffe zur Anwendung, die beispielsweise ausgewählt sind aus der Gruppe bestehend aus Betamimetika, Anticholinergika, Corticosteroiden, PDEIV-Inhibitoren, EGFR-hemmern und LTD4-Antagonisten oder zwei- oder dreifach Kombinationen davon, wie beispielsweise Kombinationen von Antcholinergika mit Betamimetika, Corticosteroiden, PDEIV-Inhibitoren, EGFR-hemmern oder LTD4-Antagonisten, oder Kombinationen von Betamimetika mit Corticosteroiden, PDEIV-Inhibitoren, EGFR-hemmern oder LTD4-Antagonisten, oder Kombinationen von Corticosteroiden mit PDEIV-Inhibitoren, EGFR-hemmern oder LTD4-Antagonisten oder Kombinationen von PDEIV-inhibitoren mit EGFR-hemmern oder LTD4-Antagonisten oder Kombinationen von EGFR-hemmern mit LTD4-Antagonisten. Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmeterol, Salmefamol, Soterenot, Sulphonterol, Tiaramide, Terbutaline, Tolubuterol, CHF-1035, HOKU-81, KUL-1248, 3-(4-{6-[2-Hydroxy-2-(4-hydroxy-3-hydroxymethyl-phenyl)-ethylamino]-hexyloxy}-butyl)-benzenesulfoneamid, 5-[2-(5,6-Diethyl-indan-2-ylamino)-1-hydroxy-ethyl]-8-hydroxy-1*H*-quinolin-2-on, 4-Hydroxy-7-[2-{[2-{[3-(2-phenylethoxy)propyl]sulphonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-Ethoxycarbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Bevorzugt sind die Betamimetika ausgewählt aus er Gruppe bestehend aus Bambuterol, Bitolterol, Carbuterol, Clenbuterol, Fenoterol, Formoterol, Hexoprenaline, Ibuterol, Pirbuterol, Procaterol, Reproterol, Salmeterol, Sulphonterol, Terbutaline, Tolubuterol, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydroxy-ethyl]-8-hydroxy-1*H*-quinolin-2-on , 4-hydroxy-7-[2-{[2-{[3-(2-phenylethoxy)propyl]sulphonyl}ethyl]-amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-on, 1-(4-amino-3-chloro-5-trifluormethylphenyl)-2-tert.-butylamino)ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-(4-ethoxycarbonylamino-3-cyano-5-fluorophenyl)-2-(tert.-butylamino)ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Besonders bevorzugte Betamimetika aind ausgewählt aus der Gruppe bestehend aus Fenoterol, Formoterol, Salmeterol, 3-(4-{6-[2-Hydroxy-2-(4-hydroxy-3-hydroxymethyl-phenyl)-ethylamino]-hexyloxy}-butyl)-benzenesulfoneamid, 5-[2-(5,6-Diethyl-indan-2-ylamino)-1-hydroxy-ethyl]-8-hydroxy-1*H*-quinolin-2-on, 1-[3-(4-Methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino]ethanol, 6-Hydroxy-8-{1-hydroxy-2-[2-(4-methoxy-phenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, gegebenenfalls in Form ihrer Racemate, Enantiomere, Diasteromere und gegebenenfalls in Form ihrer pharmakologisch verträglichen Säureadditionssalze, Solvate oder Hydrate.

Von diesen Betamimetika sind erfindungsgemäß besonders bevorzugt Formoterol, Salmeterol, 3-(4-{6-[2-Hydroxy-2-(4-hydroxy-3-hydroxymethyl-phenyl)-ethylamino]-hexyloxy}-butyl)-benzenesulfoneamide, 6-Hydroxy-8-{1-hydroxy-2-[2-(4-methoxyphenyl)-1,1-dimethyl-ethylamino]-ethyl}-4H-benzo[1,4]oxazin-3-on, 6-Hydroxy-8-{1-hydroxy-2-[2-(4-phenoxy-essigsäureethylester)-1,1-dimethyl-ethylamino]-ethyl}-4H-benzo[1,4]oxazin-3-on, 6-Hydroxy-8-{1-hydroxy-2-[2-(4-phenoxy-essigsäure)-1,1-dimethyl-ethylamino]-ethyl}-4H-benzo[1,4]oxazin-3-on, 8-{2-[1,1-Dimethyl-2-(2,4,6-trimethylphenyl)-ethylamino]-1-hydroxy-ethyl}-6-hydroxy-4H-benzo[1,4]oxazin-3-on, 6-Hydroxy-8-{1-hydroxy-2-[2-(4-hydroxy-phenyl)-1,1-dimethyl-ethylamino]-ethyl}-4H-benzo[1,4]oxazin-3-on, 6-Hydroxy-8-{1-hydroxy-2-[2-(4-isopropyl-phenyl)-1,1dimethyl-ethylamino]-ethyl}-4H-benzo[1,4]oxazin-3-on, 8-{2-[2-(4-Ethyl-phenyl)-1,1-dimethyl-ethylamino]-1-hydroxy-ethyl}-6-hydroxy-4H-benzo[1,4]oxazin-3-on, 8-{2-[2-(4-Ethoxyphenyl)-1,1-dimethyl-ethylamino]-1-hydroxy-ethyl}-6-hydroxy-4H-benzo[1,4]oxazin-3-on, 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure, 8-{2-[2-(3,4-Difluor-phenyl)-1,1-dimethyl-ethylamino]-1-hydroxy-ethyl}-6-hydroxy-4H-benzo[1,4]oxazin-3-on und 5-[2-(5,6-Diethyl-indan-2-ylamino)-1-hydroxy-ethyl]-8-hydroxy-1*H-*quinolin-2-on, gegebenenfalls in Form ihrer Racemate, Enantiomere, Diasteromere und gegebenenfalls in Form ihrer pharmakologisch verträglichen Säureadditionssalze, Solvate oder Hydrate.

Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Als Anticholinergika gelangen in den erfindungsgemäßen Verfahren bevorzugt Salze zur Anwendung, die ausgewählt sind aus der Gruppe bestehend aus Tiotropiumsalzen, Oxitropiumsalzen, Flutropiumsalzen, Ipratropiumsalzen, Glycopyrroniumsalzen und Trospiumsalzen.

In den vorstehend genannten Salzen stellen die Kationen Tiotropium, Oxitropium, Flutropium, Ipratropium, Glycopyrronium und Trospium die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt. Von besonderer Bedeutung ist das Tiotropiumbromid. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Als Antcholinergika kommen ferner bevorzugt in Betracht die nachfolgenden Verbindungen:
- 2,2-Diphenylpropionsäuretropenolester-methobromid ,
- 2,2-Diphenylpropionsäurescopinester-methobromid,
- 2-Fluor-2,2-Diphenylessigsäurescopinester-methobromid ,
- 2-Fluor-2,2-Diphenylessigsäuretropenolester-methobromid ,;
- 3,3',4,4'-Tetrafluorbenzilsäuretropenolester-Methobromid ,
- 3,3',4,4'-Tetrafluorbenzilsäurescopinester-Methobromid ,
- 4,4'-Difluorbenzilsäuretropenolester-Methobromid ,
- 4,4'-Difluorbenzilsäurescopinester-Methobromid ,
- 3,3'-Difluorbenzilsäuretropenolester-Methobromid ,
- 3,3'-Difluorbenzilsäurescopinester-Methobromid ;
- 9-Hydroxy-fluoren-9-carbonsäuretropenolester -Methobromid ;
- 9-Fluor-fluoren-9-carbonsäuretropenolester -Methobromid ;
- 9-Hydroxy-fluoren-9-carbonsäurescopinester -Methobromid ;
- 9-Fluor-fluoren-9-carbonsäurescopinester Methobromid ;
- 9-Methyl-fluoren-9-carbonsäuretropenolester Methobromid ;
- 9-Methyl-fluoren-9-carbonsäurescopinester Methobromid ;
- Benzilsäurecyclopropyltropinester-Methobromid ;
- 2,2-Diphenylpropionsäurecyclopropyltropinester -Methobromid ;
- 9-Hydroxy-xanthen-9-carbonsäurecyclopropyltropinesterMethobromid ;
- 9-Methyl-fluoren-9-carbonsäurecyclopropyltropinester-Methobromid ;
- 9-Methyl-xanthen-9-carbonsäurecyclopropyltropinester -Methobromid ;
- 9-Hydroxy-fluoren-9-carbonsäurecyclopropyltropinester -Methobromid ;
- 4,4'-Difluorbenzilsäuremethylestercyclopropyltropinester -Methobromid.
- 9-Hydroxy-xanthen-9-carbonsäuretropenolester -Methobromid ;
- 9-Hydroxy-xanthen-9-carbonsäurescopinester Methobromid ;
- 9-Methyl-xanthen-9-carbonsäuretropenolester -Methobromid ;
- 9-Methyl-xanthen-9-carbonsäurescopinester -Methobromid ;
- 9-Ethyl-xanthen-9-carbonsäuretropenolester Methobromid ;
- 9-Difluormethyl-xanthen-9-carbonsäuretropenolester -Methobromid ;
- 9-Hydroxymethyl-xanthen-9-carbonsäurescopinester -Methobromid, gegebenenfalls in Form ihrer Solvate oder Hydrate.

Im Rahmen der vorliegenden Erfindung werden unter Corticosteroiden Verbindungen verstanden, die ausgewählt sind aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α,9α-Difluoro-17α-[(2-furanylcarbonyl)oxy]-11β-hydroxy-16α-methyl-3-oxo-androsta-1,4-dien-17β-carbothionsäure (S)-fluoromethylester und 6α,9α-Difluoro-11β-hydroxy-16α-methyl-3-oxo-17α-propionyloxy-androsta-1,4-dien-17β-carbothionsäure (S)-(2-oxo-tetrahydrofuran-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Flunisolid, Beclomethason, Triamcinolon, Budesonid, Fluticason, Mometason, Ciclesonid, Rofleponid, ST-126, Dexamethason, 6α,9α-Difluoro-17α-[(2-furanylcarbonyl)oxy]-11β-hydroxy-16α-methyl-3-oxo-androsta-1,4-dien-17β-carbothionsäure (S)-fluoromethylester und 6α,9α-Ditluoro-11β-hydroxy-16α-methyl-3-oxo-17α-propionyloxy-androsta-1,4-dien-17β-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate.

Besonders bevorzugt ist das Steroid ausgewählt aus der Gruppe bestehend aus Budesonid, Fluticason, Mometason, Ciclesonid und 6α,9α-Difluoro-17α-[(2-furanylcarbonyl)oxy]-11β-hydroxy-16α-methyl-3-oxo-androsta-1,4-dien-17β-carbothionsäure (S)-fluoromethylester, gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Im Rahmen der vorliegenden Erfindung werden unter PDEIV-Inhibitoren Verbindungen verstanden, die ausgewählt sind aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*,10*bS**)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, C1-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE IV-Inhibitor ausgewählt aus der Gruppe bestehend aus Enprofyllin, Roflumilast, Ariflo (Cilomilast), AWD-12-281 (GW-842470), N-(3,5-Dichloro-1-oxo-pyridin-4-yl)-4-difluoromethoxy-3-cyclopropylmethoxybenzamid, T-440, T-2585, Arofyllin, Cis[4-Cyano-4-(3-cyclopentyloxy-4-methoxyphenyl)cyclohexan-1-carbonsäure], 2-Carbomethoxy-4-cyano-4-(3-cyclopropylmethoxy-4-difluoromethoxyphenyl)cyclohexan-1-on, Cis[4-cyano-4-(3-cyclopropylmethoxy-4-difluoromethoxyphenyl)cyclohexan-1-ol], PD-168787, Atizoram, V-11294A, C1-1018, CDC-801, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugt ist der PDE IV-Inhibitor ausgewählt aus der Gruppe bestehend aus Roflumilast, Ariflo (Cilomilast), AWD-12-281 (GW-842470), Arofyllin, 2-Carbomethoxy-4-cyano-4-(3-cyclopropylmethoxy-4-difluoromethoxyphenyl)cyclohexan-1-on, Cis [4-cyano-4-(3-cyclopropylmethoxy-4-difluoromethoxyphenyl)cyclohexan-1-ol], Atizoram, Z-15370 , 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H* pyrazolo[3,4-c]-1,2,4-triazolo[4,3-a]pyridin, wobei Roflumilast, Z-15370 und AWD-12-281 besondere Bedeutung zukommt, gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die o.g. PDEIV-inhibitoren gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Im Rahmen der vorliegenden Erfindung werden unter LTD4-Antagonisten Verbindungen verstanden, die ausgewählt sind aus der Gruppe bestehend aus Montelukast, 1-(((R)-(3-(2-(6,7-Difluoro-2-quinolinyl)ethenyl)phenyl)-3-(2-(2- hydroxy-2-propyl)phenyl)thio)-methylcyclopropan-essigsäure, 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure, Pranlukast, Zafirlukast, [2-[[2-(4-tert-Butyl-2-thiazolyl)-5-benzofuranyl]oxymethyl]phenyl]essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Bevorzugt ist der LTD4-Antagonist ausgewählt aus der Gruppe bestehend aus Montelukast, Pranlukast, Zafirlukast, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Besonders bevorzugt ist der LTD4-Antagonist ausgewählt aus der Gruppe bestehend aus Montelukast, Pranlukast, Zafirlukast, MCC-847 (ZD-3523), MN-001 und MEN-91507 (LM-1507), wobei Montelukast , Pranlukast und Zafirlukast besonders bevorzugt sind, gegebenenfalls in Form ihrer Racemate, Enantiomere oder Diastereomere, gegebenenfalls in Form ihrer pharmakoligisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Unter Salzen oder Derivaten zu deren Bildung die LTD4-antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Im Rahmen der vorliegenden Erfindung werden unter EGFR-Hemmem Verbindungen verstanden, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(*R*)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((*R*)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((*R*)-6-methyl-2-oxo-morpholin-4-yl)-1-oxo-2-buten-1-yl]amino}-7-[(*S*)-(tetrahydrofuran-3-yl)oxy]-chinazolin, 4-[(3-Chlor-4-fluor-phenyl)amino]-6-{[4-((*R*)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((*S*)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(*R*)-(1-Phenyl-ethyl)amino]-6-{[4-(N,N-bis-(2-methoxyethyl)-amino)-1-oxo-2-buten-1-yl]amino}-7-cyclopropylmethoxy-chinazolin, 4-[(*R*)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(*R*)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(*R*)-(1-Phenyl-ethyl)amino]-6-({4-[N-(tetrahydropyran-4-yl)-N-methyl-amino]-1-oxo-2-buten-l-yl}amino)-7-cyclopropylmethoxy-chinazolin, 4-[(3-Chlor-4-fluorphenyl)amino]-6-{[4-(N,N-dimethylamino)-1-oxo-2-buten-1-yl]ammo}-7-((*R*)-tetrahydrofuran-3-yloxy)-chinazolin, 4-[(3-Chlor-4-fluorphenyl)amino]-6-{[4-(N,N-dimethylamino)-1-oxo-2-buten-yl]amino}-7-((*S*)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(*R*)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(methoxymethyl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluorphenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluorphenyl)amino]-6-(cis-4-{N-[(tetrahydropyran-4-yl)carbonyl]-N-methyl-amino}-cyclohexan-l-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluorphenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxyacetyl)-piperidin-4-yloxy]-7-(2-methoxy-ethoxy)-chinazolin, 4-[(3-Chlor-4-fluorphenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluorphenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxyacetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazoiln, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin , 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin , 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Bevorzugte EGFR-Hemmer sind ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethyl)amino)-1-oxo-2-buten-1-yl]amino}-7-cyclopentyloxy-chinazolin, 4-[(R)-(1-Phenyl-ethyl)amino]-6-{[4-(N,N-bis-(2-methoxyethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cydopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin , 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl }-furan-2-yl)chinazolin , 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyt)amino]-6-{1-[(morpholin-4-yl)carbonyl]-piperidin-4-yloxy}-7-methoxy-chinazolin, 4-[(3-Chlor-4-fluorphenyl)amino]-6-{ 1-[(methoxymethyl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluorphenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin, 4-[(3-Chlor-4-fluorphenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonyl-piperidin-4-yloxy)-7-(2-methoxy-ethoxy)-chinazolin , 4-[(3-Chlor-4-fluor-phenyl)amino]-6-[1-(2-methoxy-acetyl)-piperidin-4-yloxy]-7-(2-methoxy-ethoxy)-chinazolin, 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4- [(3-Chlor-4-fluor-phenyl)amino]-6-(cis-4-{N-[piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxyacetyl)-N-methyl-amino]-cyclohexan-1-yloxy}-7-methoxy-chinazolin, 4-[(3-Ethinyl-phenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin , 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin , 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin, 4-[(3-Chlor-4-fluor-phenyl)amino-6-(1-cyano-piperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Besonders bevorzugt gelangen im Rahmen der vorliegenden Erfindung diejenigen EGFR-Hemmer zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluorphenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxyethyl)-N-methyl-amino]-1-oxo-2-buten-1-yl}amino)-7-cyclopentyloxy-chinazolin, 4-[(3-Chlor-4-fluorphenyl)amino]-6-{[4-N,N-dimethylamino)-1-oxo-2-buten-1-yl]amino}-7-[(R)-(tetrahydrofuran-2-yl)methoxy]-chinazolin, 4-[(3-Ethinyl-phenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-[(R)-(1-Phenylethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluorphenyl)amino]-6-(tetrahydropyran-3-yloxy)-7-methoxy-chinazolin, 4-[(3-Chlor-4-fluorphenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin,
4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxyacetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidm-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin , 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluorphenyl)amino]-6-[trans-4-(N-methansulfonyl-N-methyl-amino)-cyclohexan-1-yloxy]-7-methoxy-chinazolin , 4-[(3-Chlor-4-fluor-phenyl)amino]-6-(trans-4-dimethylamino-cyclohexan-1-yloxy)-7-methoxy-chinazolin , 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydroftuan-2-yl)methoxy]-chinazolin, 4-[(3-Chlor-4-fluorphenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugt sind als EGFR-Hemmer diejenigen Verbindungen, die ausgewählt sind aus der Gruppe bestehend aus
- 4-[(3-Chlor-4-fluorphenyl)amino]-6-{[4-(morpholin-4-yl)-1-oxo-2-buten-1-yl]amino}-7-cyclopropylmethoxy-chinazolin,
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin,
- 4-[(3-Chlor-4-fluor-phenyl)amino]-6-[2-((S)-6-methyl-2-oxo-morpholin-4-yl)-ethoxy]-7-methoxy-chinazolin,
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
- 4-[(3-Ethinyl-phenyl)amino]-6,7-bis-(2-methoxy-ethoxy)-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Ethinyl-phenyl)amino]-6-{[4-(5,5-dimethyl-2-oxo-morpholin-4-yl)-1-oxo-2-buten-1-yl]amino}-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-{1-[(morpholin-4-yl)carbonyl]-pipendin-4-yl-oxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-l-yloxy)-7-methoxy-chinazolin,
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)anino]-6-(1-methansulfonyl-piperidin-4-yloxy)-7-methoxy-chinazolin und
- 4-[(3-Chlor-4-fluor-phenyl)amino]-6-(1-cyano-piperidin-4-yloxy)-7-methoxy-chinazolin,
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die EGFR-Hemmer gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Überraschenderweise hat sich gezeigt, dass Pulverinhalativa, die die nach der erfindungsgemäßen Vorgehensweise erhältliche Laktose als Hilfsstoff enthalten, nicht nur einen erhöhten inhalierbaren Anteil aufweisen, sondern dass vor allem Formulierungen basierend auf dieser Erfindung auch eine verbesserte "Robustheit" zeigen.

Ein weiterer Aspekt betrifft daher ferner Inhalationspulver, die neben einem oder mehreren der vorstehend genannten Wirkstoffe als Hilfsstoff die erfindungsgemäß erhältliche Laktosemischung enthalten.

Ferner betrifft die vorliegende Erfindung die Verwendung des nach dem erfindungsgemäßen Verfahren erhältlichen Laktose zur Herstellung eines Arzneimittels zur Behandlung von Krankheiten, in denen die inhalative Applikation von Wirkstoffhaltigen Arzneimitteln einen therapeutischen Nutzen entfalten kann. Ferner betrifft die vorliegende Erfindung die Verwendung des nach dem erfindungsgemäßen Verfahren erhältlichen Laktosemischung zur Herstellung eines wirkstoffhaltigen Inhalationspulvers.

## Patentansprüche

1. Verwendung einer Laktosemischsung als Hilfsstoff bei der Herstellung eines wirkstoffhaltigen Inhalationspulvers
wobei die Laktosemischung hergestellt wird durch ein Verfahren, bei dem in einem ersten Schritt eine Trägerlaktose mit einer mittleren Teilchengröße*, von X₅₀ = 17-50µm einer Laktosefraktion mit einer geringeren mittleren Teilchengröße* von X₅₀< 25µm zugemischt wird, wobei die Laktosefraktion mit der geringeren mittleren Teilchengröße einen hohen Anteil amorphes Laktose enthält, und in einem Zweiten Schritt die so erhaltene Laktosemischung einer Relation Luftfeuchte von wenigstens 85% r.F. bei einer Temperatur von 20°C-40°C für einen Zeitraum von wenigstens 4 Stunden ausgesetzt wird, und in einem dritten Schritt die erhaltene Laktosemischung einem Trocknungsschritt ausgesetzt wird, wobei die Laktose einer Luftfeuchte kleiner 20% r.F. unter Erhöhung der Temperatur für einen Zeitraum von mindestens 1 Stunde ausgesetzt wird.
*gemessen mit einem Laserdiffraktometer nach der Trockendispersionsmethode

2. Verwendung der Lacktosemischung nach Anspruch 1
**dadurch gekennzeichnet, dass** die nach dem ersten Schritt erhaltene Laktosemischung einer relativen Feuchte von wenigstens 90% r.F. ausgesetzt wird.

3. Verwendung der Laktosemischung
nach einem der Ansprüche 1 bis 2,
**dadurch gekennzeichnet, dass** die nach dem ersten Schritt erhaltene Laktosemischung im zweiten Schritt
a) derart bereitgestellt wird, dass eine möglichst große Pulverbett-Oberfläche ausgebildet wird,
b) dass sie sodann den in Anspruch 1 genannten Bedingungen des Konditionierungsschritts ausgesetzt wird:
c) und dann bei dem Trocknungsschritt gemäss Anspruch 1 eine Reduzierung der relativen Luftfeuchte, welcher das Laktose-Muster ausgesetzt wird, erfolgt.

4. Verwendung der Laktosemischung
nach einem der Ansprüche 1 bis 3,
**dadurch gekennzeichnet, dass** der Wirkstoff ausgewählt ist aus der Gruppe bestehend aus Betamimetika, Anticholinergika, Corticosteroiden, PDEIV-Inhibitoren, EGFR-hemmern und LTD4-Antagonisten oder zwei- oder dreifach Kombinationen davon.

## Claims

1. Use of a lactose mixture as an excipient in the manufacture of an inhalable powder containing an active substance, wherein the lactose mixture is prepared by a process wherein, in a first step, a carrier lactose with an average particle size of x₅₀ = 17 - 50 µm, measured with a laser diffractometer using the dry dispersion method, is mixed with a lactose fraction with a small average particle size of x₅₀ < 25µm, measured with a laser diffractometer using the dry dispersion method, the lactose fraction with the smaller average particle size containing a high proportion of amorphous lactose, and in a second step the lactose mixture thus obtained is exposed to a relative humidity of at least 85% r.h. at a temperature of 20°C-40°C for a period of at least 4 hours, and in a third step the lactose mixture obtained is subjected to a drying step, the lactose being exposed to a relative humidity of less than 20% r.h. while the temperature is increased for a period of at least 1 hour.

2. Use of the lactose mixture according to claim 1, **characterised in that** the lactose mixture obtained according to the first step is exposed to a relative humidity of at least 90% r.h.

3. Use of the lactose mixture according to one of claims 1 and 2, **characterised in that** in the second step the lactose mixture obtained according to the first step
a) is prepared in such a way as to form the largest possible surface area for the powder bed,
b) it is then exposed to the conditions of the treatment step recited in claim 1,
c) and then, in the drying step according to claim 1, the relative humidity to which the lactose sample is exposed is reduced.

4. Use of the lactose mixture according to one of claims 1 to 3, **characterised in that** the active substance is selected from among the betamimetics, anticholinergics, corticosteroids, PDEIV-inhibitors, EGFR-inhibitors and LTD4-antagonists or double or triple combinations thereof.

## Revendications

1. Utilisation d'un mélange de lactoses comme adjuvant lors de la production d'une poudre pour inhalation contenant un principe actif où le mélange de lactoses est produit par un procédé dans lequel, dans une première étape, un lactose vecteur d'une taille de particule moyenne, mesurée avec un diffractomètre laser selon la méthode de dispersion sèche, de x₅₀ = 17 - 50 µm est mélange avec une fraction de lactose de plus petite taille de particule moyenne, mesurée avec un diffractomètre laser selon la méthode de dispersion sèche, de x₅₀ < 25 µm, où la fraction de lactose de plus petite taille de particule moyenne contient une grande proportion de lactose amorphe, et, dans une deuxième étape, le mélange de lactoses ainsi obtenu est exposé à une humidité de l'air relative d'au moins 85 % H. R. à une température de 20°C - 40°C pendant une durée d'au moins 4 heures, et, dans une troisième étape, le mélange de lactoses obtenu est exposé à une étape de séchage où le lactose est exposé à une humidité de l'air inférieure à 20 % H. R. avec augmentation de la température pendant une durée d'au moins 1 heure.

2. Utilisation du mélange de lactoses selon la revendication 1 **caractérisée en ce que** le mélange de lactoses obtenu après la première étape est exposé à une humidité relative d'au moins 90 % H.R.

3. Utilisation du mélange de lactoses selon l'une des revendications 1 à 2 **caractérisée en ce que** le mélange de lactoses obtenu après la première étape
a) est préparé dans la deuxième étape de telle manière qu'une surface de lit de poudre la plus grande possible est formée,
b) **en ce qu'**il est ensuite exposé aux conditions de l'étape de conditionnement citées dans la revendication 1 ;
c) après quoi, dans l'étape de séchage selon la revendication 1, a lieu une réduction de l'humidité de l'air relative à laquelle est exposé l'échantillon de lactose.

4. Utilisation du mélange de lactoses selon l'une des revendications 1 à 3 **caractérisée en ce que** le principe actif est choisi dans le groupe consistant en les bêtamimétiques, les anticholinergiques, les corticostéroïdes, les inhibiteurs de PDEIV, les inhibiteurs de EGFR et les antagonistes de LTD4 ou leurs combinaisons doubles ou triples.
